# EUROPEAN PATENT APPLICATION

(11) **EP 1 616 861 A2**
(43) Date of publication of application: **18.01.2006**
(21) Application number: 05011646.6
(22) Date of filing: 31.05.2005
(51) Int. Cl.: C07D 211/22, A61K 31/4515, A61P 43/00, A61P 37/08, A61P 11/08

(54) **A process for the preparation of keto compounds**

(30) Priority: 15.06.2004 IT MI20041191; 13.07.2004 IT MI20041397
(71) Applicant: Dipharma S.p.A., 33036 Mereto di Tomba (Udine) (IT)
(72) Inventor: Castaldi, Graziano, 28072 Briona (NO) (IT); Barreca, Giuseppe, 23874 Montevecchia (LC) (IT); Tarquini, Antonio, 15057 Tortona (AL) (IT)
(74) Representative: Minoja, Fabrizio

(57) **Abstract**

A process for the preparation of 4-[1-oxo-4-[4-(hydroxydiphenylmethyl)-1-piperidinyl]butyl]-α,α-dimethylbenzeneacetic acid, useful as an intermediate in the preparation of fexofenadine.

## Description

### FIELD OF THE INVENTION

The present invention relates to a novel process for the preparation of 4-[1-oxo-4-[4-(hydroxydiphenylmethyl)-1-piperidinyl]butyl]-α,α-dimethylbenzeneacetic acid and its use in the preparation of fexofenadine.

### TECHNOLOGICAL BACKGROUND

A number of processes for the preparation of fexofenadine are known, for example those disclosed in WO 93/21156, WO 97/22344 and WO 97/23213, characterized by a high number of steps. None of the known processes involves a convergent approach, but the construction of the molecule through stepwise introduction of the different functionalities starting from α,α-dimethylbenzeneacetic acid. An alternative route is described by Kawai S. et al. in J. Org. Chem. 1994, 59, 2620-2622, but it has some disadvantages which prevent its industrial application. A key step in said synthetic route is, in fact, the hydration of the alkyne bond in the carboxymethyl ester of formula (A) to give the respective keto derivative of formula (B).

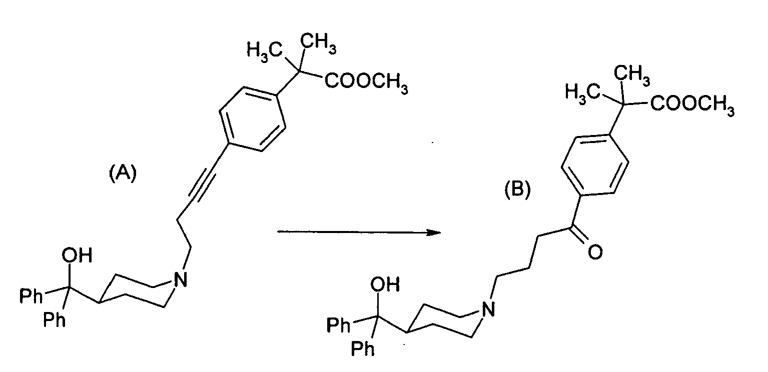

This is however accompanied by formation of by-products, which are difficult to remove from the final product. The paper by Kawai S. et al. describes in fact the subsequent purification of the ketone of formula (**B**) by silica gel chromatography. It is well-known that this technique is hardly suitable for the production of large amounts of the product, so that this process is not industrially applicable. The problem of the formation of by-products was solved by EP 1260505, in which the hydration is carried out using a catalyst based on platinum, palladium or ruthenium, optionally in the presence of ligands. The very high cost of these catalysts, however, negatively affects the final costs. Therefore, there still is the problem of hydrating the alkyne bond to obtain the corresponding keto compound, avoiding the formation of hardly removable by-products as well as the increase in industrial production costs.

### SUMMARY OF THE INVENTION

It has now surprisingly been found that the reaction between the alkyne of formula (**A**), in the form of the free carboxylic acid, and a solution of HgO in H₂SO₄ provides the respective keto derivative in industrial yields, without need for expensive catalysts as well as complex, time-consuming purification operations.

### DETAILED DISCLOSURE OF THE INVENTION

The object of the invention is a process for the preparation of a compound of formula (**I**) 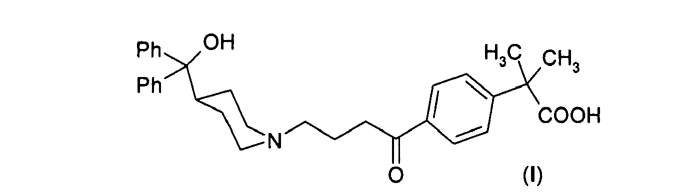 comprising the reaction of a compound of formula (**II**) 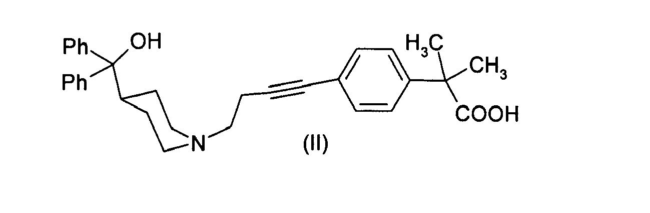 with a sulfuric acid aqueous solution, in the presence of mercury(**II**) oxide, in a C₁-C₄ alkanol.

A C₁-C₄ alkanol, in which the alkyl moiety can be straight or branched, is preferably methanol or ethanol, in particular methanol.

The alkyne of formula (**II**) is reacted in the form of a solution in a C₁-C₄ alkanol, as defined above, in a concentration approx. from 10 to 30%, preferably from 15 to 25% weight/volume.

The sulfuric acid aqueous solution is typically a solution approx. from 30 to 50%, preferably from 35 to 45% weight/volume.

The reaction is carried out contacting the alkanol solution of the alkyne of formula (**II**) with the sulfuric acid aqueous solution in molar amounts of sulfuric acid to alkyne of formula (**II**) ranging approx. from 0.8 to 1.2, preferably from 0.9 to 1.1. The resulting solution is added with HgO in molar amounts to the alkyne of formula (**II**) ranging approx. from 0.01 to 0.05, preferably from 0.02 to 0.04. The reaction is carried out at a temperature ranging approx. from 20 to 60°C, preferably from 30 to 50°C.

After completion of the reaction, the reaction mixture is alkalinised with a sodium hydroxide methanolic solution, the resulting sodium salt of the ketone of formula (**I**) is transformed into the corresponding free acid by treatment with a mineral acid, such as hydrochloric, sulfuric or phosphoric acid, or an organic acid, for example acetic, methanesulfonic or oxalic acid.

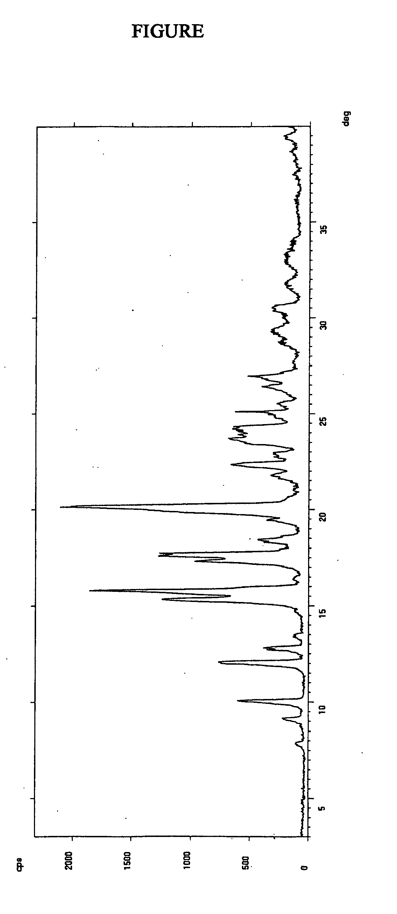

The resulting ketone of formula (**I**): 4-{[4-(4-hydroxydiphenylmethyl)-1-piperidinyl]-1-oxobutyl}-α,α-dimethylbenzeneacetic acid, has crystalline structure having X-ray diffraction spectrum (X-ray powder diffraction, XRPD) substantially as reported in the Figure, the more intense diffraction peaks being observed at 10.05; 12.03; 15.33; 15.78; 17.34; 17.64; 20.13 and 23.67 in 2θ, and this is a further object of the invention. The XRPD spectrum was recorded with an APD 2000 automatic diffractometer θ/θ for powders and liquids (available from Ital-Structures), under the following operative conditions: CuKα radiation (λ = 1.5418 A), scanning with angular pass of 0.03° for a time of 1 sec".

The comparative data reported in the following table show the advantages provided by the process of the present invention compared with that described by Kawai S. et al.

**Table**

| Starting alkyne | Starting mols | Keto compound | Mols of keto compound | % Yield |
|---|---|---|---|---|
| Compound (**A**) | 1.18 | Compound (**B**) | 0.885 | 75 |
| Compound (**II**) | 1.18 | Compound (**I**) | 1.062* | 90* |
| | | | 0.968** | 82** |

| | | | | |
|---|---|---|---|---|
| (*): values obtained by titration of the final reaction mixture. | | | | |
| (**): values concerning the isolated product. | | | | |

Since the formed by-products are difficult to remove, when the reaction is carried out according to Kawai S. et al., the purification of the ketone of formula (**B**) is carried out by silica gel chromatography (1:2 → 3:1 ethyl acetate - hexane, then 15:1 CH₂-Cl₂ - methanol, v/v), as described in the same paper. On the other hand, the keto compound of formula (I), after work up, is separated by simple precipitation. Moreover, the amount of HgO used according to the process of the invention is about 1/10 that used by Kawai S. et al., thereby reducing the environmental impact.

The keto compound of formula (**I**) can be subsequently reduced to obtain fexofenadine of formula (**III**) 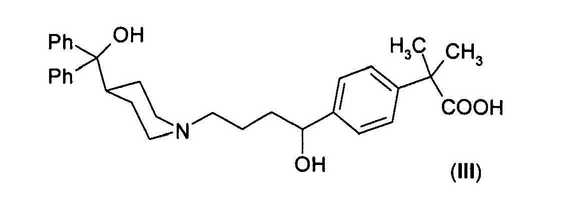
by means of known procedures, for example, by a process comprising the reduction with a reducing agent, such as sodium borohydride, potassium borohydride, sodium cyanoborohydride or tetramethylammonium borohydride, in a suitable alkanol, such as methanol, ethanol, isopropanol, n-butanol or mixtures thereof with water, at a temperature ranging from about 0°C to the reflux temperature of the reaction mixture. If desired, fexofenadine can then be converted into a salt, for example the hydrochloride, according to known methods.

Therefore, the present invention also relates to a process for the preparation of fexofenadine, or a pharmaceutically acceptable salt thereof, comprising the reduction of a keto compound of formula (**I**) 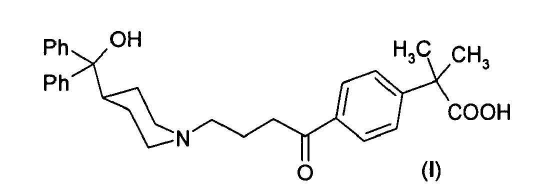
and, if desired, the conversion into a salt thereof, such as the hydrochloride, characterized in that the compound of formula (**I**) is obtained by reaction of a compound of formula (**II**) 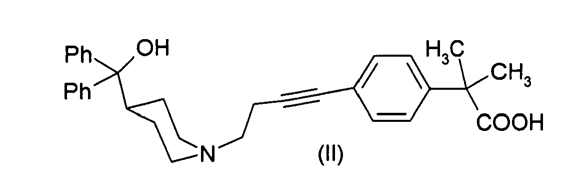
with a sulfuric acid aqueous solution, in the presence of mercury(**II**) oxide, in a C₁-C₄ alkanol, as herein described.

The following example illustrates the invention.

### Example: Synthesis of 4-{[4-(4-hydroxydiphenylmethyl)-1-piperidinyl]-1-oxobutyl}-α,α -dimethylbenzeneacetic acid; (I)

A four-necked three litres flask, equipped with stirrer, thermometer, condenser and kept under nitrogen, is loaded with 353 g of 4-{[4-(4-hydroxydiphenylmethyl)-1-piperidinyl]-1-butynyl}-α,α-dimethylbenzeneacetic acid and 1790 ml of methanol. A solution of 72 g of 96% w/w sulfuric acid in 185 g of water is prepared in a 500 ml flask and added with 4.1 g of mercury(II) oxide under stirring. The resulting suspension of 4-{[4-(4-hydroxydiphenylmethyl)-1-piperidinyl]-1-butynyl}-α,α-dimethylbenzeneacetic acid in methanol is added with the mercury sulfate aqueous solution prepared above. The resulting solution is heated at about 40°C under stirring, keeping this temperature until completion of the reaction. (The yield being now above 90%). A solution of sodium hydroxide scales (86 g) in 430 ml of methanol is prepared and added with the reaction mixture at room temperature: temperature spontaneously raises, to obtain a suspension. The mixture is refluxed and 66 g of glacial acetic acid are dropped therein. After adding approx. 30% of the acid, crystallization is started with the previously obtained pure keto compound (I), and the addition is completed. After that, the mixture is refluxed for about 15-20 minutes, then cooled in approx. 2 hours at 25-30°. The mixture is left at this temperature for approx. an hour and the solid is filtered and washed with methanol (2x100 ml). The resulting solid is further purified from the inorganic salts by hot trituration in 950 ml of water and filtration at 60-65°C. After washing with 100 ml x 2 of water and washing with 100 ml x 2 of methanol, the product is dried. 281 g of compound (I) in the crystalline form, having an XRPD spectrum substantially as reported in the Figure, are obtained; (purity above 99.5%; yield 82%).

## Claims

1. A process for the preparation of a compound of formula (**I**) 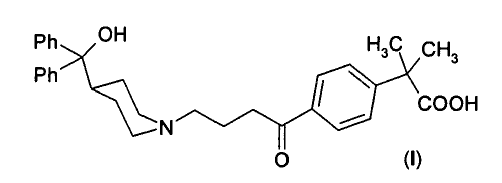 comprising the reaction of a compound of formula (**II**) 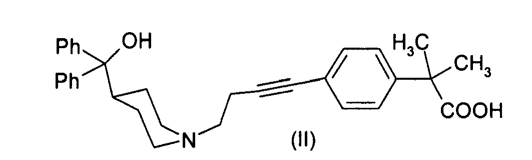 with a sulfuric acid aqueous solution, in the presence of mercury(II) oxide, in a C₁-C₄ alkanol.

2. A process as claimed in claim 1, wherein the alkanol is methanol or ethanol.

3. A process as claimed in claims 1 or 2, wherein the concentration of the compound of formula (**II**) in the alkanol ranges from 10 to 30% weight/volume.

4. A process as claimed in claims 1 or 3, wherein the concentration of the sulfuric acid aqueous solution ranges from 30 to 50% weight/volume.

5. A process as claimed in claims 1 or 4, wherein the molar amount of sulfuric acid to compound of formula (**II**) ranges from 0.8 to 1.2.

6. A process as claimed in claim 5, wherein the molar amount of sulfuric acid to compound of formula **(II)** ranges from 0.9 to 1.1.

7. A process as claimed in claims 1 or 6, wherein the molar amount of mercury(II) oxide to compound of formula (**II**) ranges from 0.01 to 0.05.

8. A process as claimed in claim 7, wherein the molar amount of mercury(II) oxide to compound of formula (**II**) ranges from 0.02 to 0.04.

9. 4-{[4-(4-Hydroxydiphenylmethyl)-1-piperidinyl]-1-oxobutyl}-α,α-dimethylbenzeneacetic acid in the crystalline form.

10. A process for the preparation of fexofenadine, or a pharmaceutically acceptable salt thereof, comprising the reduction of a keto compound of formula (**I**) 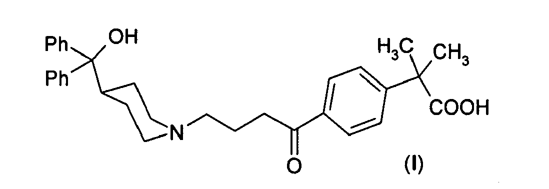 and, if desired, the conversion into a salt thereof, **characterized in that** the compound of formula (**I**) is obtained by reaction between a compound of formula (**II**) 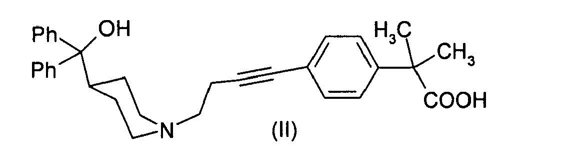 and a sulfuric acid aqueous solution, in the presence of mercury(II) oxide, in a C₁-C₄ alkanol, as claimed in each of claims 1 to 8.

11. A process as claimed in claim 10, wherein the fexofenadine salt is the hydrochloride.

12. A crystalline form of the acid of claim 9, having an XRPD spectrum substantially as reported in the Figure.

13. A crystalline form as claimed in claim 12, having an XRPD spectrum in which the more intense diffraction peaks are observed at 10.05; 12.03; 15.33; 15.78; 17.34; 17.64; 20.13 and 23. 67 in 20.
